(19) Europäisches Patentamt
European Patent Office
Office européen des brevets

(11) **EP 2 233 098 B1**

(12) **EUROPEAN PATENT SPECIFICATION**

(45) Date of publication and mention
of the grant of the patent:
**07.01.2015   Bulletin 2015/02**

(51) Int Cl.:
***A61B 18/00*** *(2006.01)*       ***A61B 18/18*** *(2006.01)*

(21) Application number: **10157500.9**

(22) Date of filing: **24.03.2010**

(54) **Apparatus for tissue sealing**

Vorrichtung zum Versiegeln von Gewebe

Appareil pour l'étanchéité de tissus

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **24.03.2009   US 410195**

(43) Date of publication of application:
**29.09.2010   Bulletin 2010/39**

(60) Divisional application:
**13169462.2 / 2 641 559
14194675.6**

(73) Proprietor: **Covidien LP
Mansfield, MA 02048 (US)**

(72) Inventors:
• **Nau, William H.
Longmont, CO 80504 (US)**
• **Rossetto, Francesca
Longmont, CO 80504 (US)**

(74) Representative: **Hoffmann Eitle
Patent- und Rechtsanwälte PartmbB
Arabellastraße 30
81925 München (DE)**

(56) References cited:
WO-A1-2008/044012       US-A- 4 315 510
US-A- 6 152 923       US-A1- 2004 082 952
US-A1- 2008 215 050       US-A1- 2009 015 832
US-B1- 6 471 696

## Description

## BACKGROUND

### Technical Field

The present disclosure relates to forceps for sealing various types of tissue. More particularly, the present disclosure relates to open, laparoscopic or endoscopic forceps that utilize microwave energy to seal tissue.

### Description of the Related Art

In many surgical procedures, body vessels, e.g., blood vessels, ducts, adhesions, fallopian tubes, etc. are sealed to defunctionalize or close the vessel. Traditionally, staples, clips or sutures have been used to close a body vessel. However, these traditional procedures often leave foreign body material inside a patient. In an effort to reduce foreign body material left within the patient and to more effectively seal the body vessel, energy techniques that seal by heat processes have been employed.

A forceps is particularly useful for sealing tissue and vessels since forceps utilizes mechanical action to constrict, grasp, dissect and/or clamp tissue. Current vessel sealing procedures utilize heat treatment to heat and desiccate tissue causing closure and sealing of the body vessel. In addition, forceps allow for control of the applied pressure to the tissue. The combination of heating and applied pressure provides a uniform, controllable seal and that is capable of providing such a seal with minimum collateral damage to body tissue.

US 6,152,923 discloses a bipolar forceps having the features of the first part of claim 1 and further including a pair of multipronged forceps pivotally attached in opposing relation.

## SUMMARY

The present invention is defined in claim 1. It provides a microwave forceps for sealing tissue. The forceps includes a shaft member having an end effector assembly disposed at a distal end thereof. The end effector assembly includes opposing jaw members movable from a first position in spaced relation relative to one another to at least one subsequent position wherein the jaw members cooperate to grasp tissue therebetween. Each of the jaw members includes a sealing surface, wherein one of the sealing surfaces includes one or more microwave antenna assemblies coupled to a source of microwave energy.

The microwave antenna assembly includes: a slot antenna having a substantially rectangular slot defined therethrough, the rectangular slot having a first longitudinal side coupled to a ground reference of the microwave energy source and a second longitudinal side coupled to an active element of the microwave energy source.

## BRIEF DESCRIPTION OF THE DRAWINGS

Various embodiments of the present disclosure are described herein with reference to the drawings wherein:

Fig. 1 is a perspective view of a tissue sealing system including a forceps and an energy generator according to one embodiment of the present disclosure;
Fig. 2 is a cross-sectional view of a distal end of the forceps of Fig. 1;
Figs. 3A-3B are views of a microwave end effector assembly according to a non-embodiment of the present invention;
Figs. 4A-4B are views of a microwave end effector assembly according to another non-embodiment of the present invention;
Figs. 5A-5B are views of a microwave end effector assembly according to another non-embodiment of the present invention; and
Figs. 6A-6C are views of a microwave end effector assembly according to an embodiment of the present invention.

## DETAILED DESCRIPTION

Various embodiments of the present disclosure are described hereinbelow with reference to the accompanying drawings. Well-known functions or constructions are not described in detail to avoid obscuring the present disclosure in unnecessary detail. Those skilled in the art will understand that the present disclosure may be adapted for use with either an endoscopic instrument or an open instrument; however, different electrical and mechanical connections and considerations apply to each particular type of instrument. The novel aspects with respect to vessel and tissue sealing are generally consistent with respect to both the open and endoscopic designs. In the drawings and in the description that follows, the term "proximal", as is traditional, will refer to the end of the forceps that is closer to the user, while the term "distal" will refer to the end of the forceps that is further from the user.

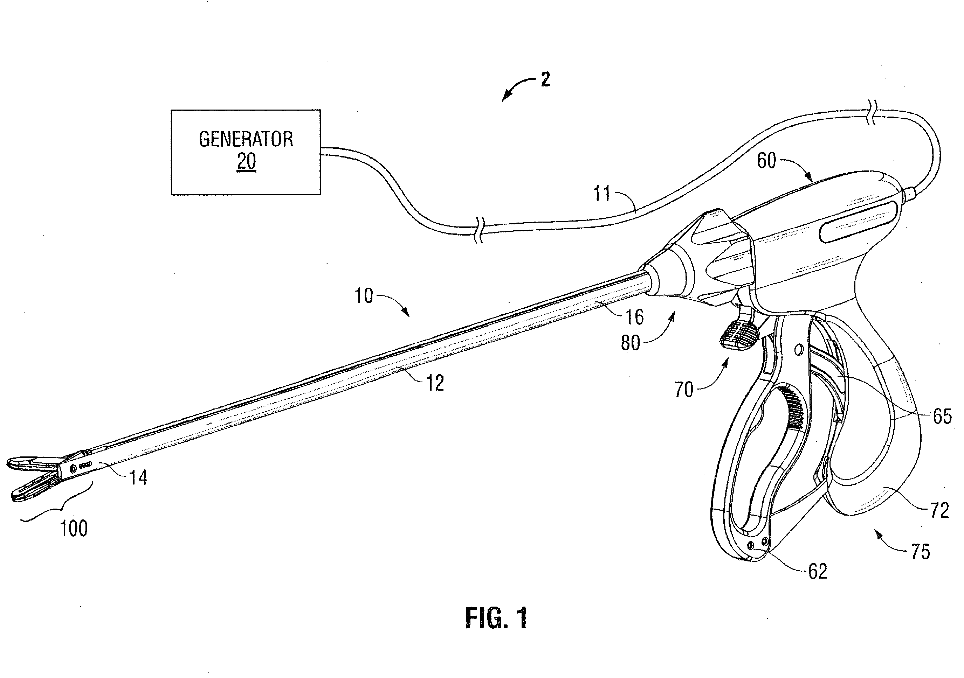

Referring now to Fig. 1, a tissue sealing system 2 according to the present disclosure is shown including a forceps 10 coupled to a generator 20. The forceps 10 is adapted to seal tissue using microwave energy. The generator 20 may be configured to output various types of microwave energy (e.g., from about 300 MHz to about 10,000 MHz).

The forceps 10 is coupled to the generator 20 via a cable 11 adapted to transmit energy and control signals therebetween. Various embodiments of the forceps 10 utilizing the aforementioned types of energy are discussed in more detail below.

The forceps 10 is configured to support an end effector assembly 100. Forceps 10 typically includes various conventional features (e.g., a housing 60, a handle assembly 75, a rotating assembly 80, a trigger assembly 70) that enable forceps 10 and end effector assembly 100 to mutually cooperate to grasp, seal and, if warranted, divide tissue. Forceps 10 generally includes housing 60 and handle assembly 75, which includes moveable handle 62 and handle 72 that is integral with housing 60. Handle 62 is moveable relative to handle 72 to actuate end effector assembly 100 to grasp and treat tissue. Forceps 10 also includes a shaft 12 that has distal end 14 that mechanically engages end effector assembly 100 and proximal end 16 that mechanically engages housing 60 proximate rotating assembly 80 disposed at the distal end of housing 60. Rotating assembly 80 is mechanically associated with shaft 12. Movement of rotating assembly 80 imparts similar rotational movement to shaft 12 which, in turn, rotates end effector assembly 100.

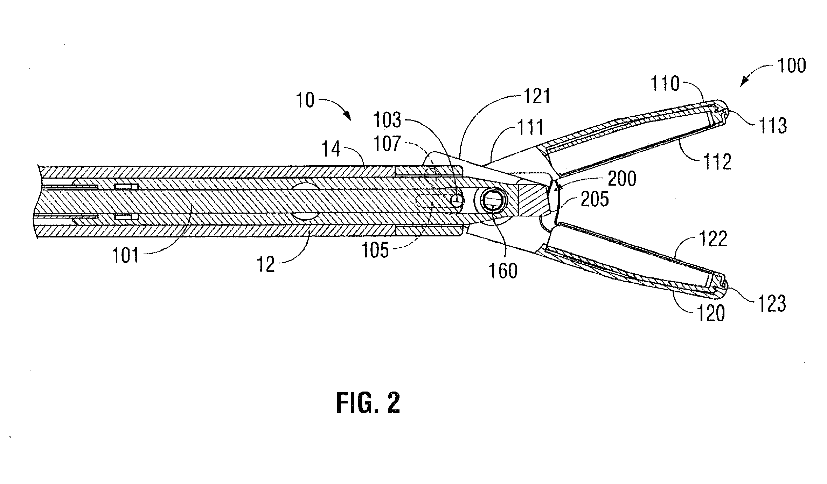

Referring to Fig. 2, the end effector assembly 100 includes two jaw members 110 and 120 having proximal ends 111, 121 and distal ends 113, 123. Jaw members 110 and 120 are pivotable about a post 160 and are movable from a first position wherein jaw members 110 and 120 are spaced relative to another, to a second position wherein jaw members 110 and 120 are closed and cooperate to grasp tissue therebetween. As discussed in more detail below, the end effector assembly 100 may be adapted for use with various energy sources.

The shaft 12 houses a pushrod 101 that is operatively coupled to the movable handle 62 such that when the handle 62 is moved relative to the handle 72 the pushrod 101 moves longitudinally, either proximally or distally within the shaft 12. The pushrod 101 includes a push pin 103 disposed at the distal end 16 of shaft 12. Each of the jaw members 110 and 120 includes a slot 105 and 107, respectively, disposed at the proximal ends thereof. The slots 105 and 107 are in mechanical cooperation with the push pin 103, which is adapted to move within the slots 105 and 107, The pin 103 and slots 105 and 107 operate as a cam-follower mechanical linkage. Motion of the pushrod 101 causes the pin 103 to slide within respective slots 105 and 107. The slots 105 and 107 may be angled with respect to the distal ends of the jaws members 110 and 120 such that the members 110 and 120 move either toward or away from each other as the pushrod 101 is moved longitudinally in a proximal or distal direction, respectively.

The forceps 10 also includes a trigger assembly 70 that advances a knife 200 disposed within the end effector assembly 100. Once a tissue seal is formed, the user activates the trigger assembly 70 to separate the tissue along the tissue seal. Knife 200 includes a sharpened edge 205 for severing the tissue held between the jaw members 110 and 120 at the tissue sealing site.

Each jaw member 110 and 120 includes a sealing surface 112 and 122, respectively, disposed on an inner-facing surface thereof. Sealing surfaces 112 and 122 cooperate to seal tissue held therebetween upon the application of energy. Sealing surfaces 112 and 122 are connected to generator 20 that communicates energy through the tissue held therebetween.

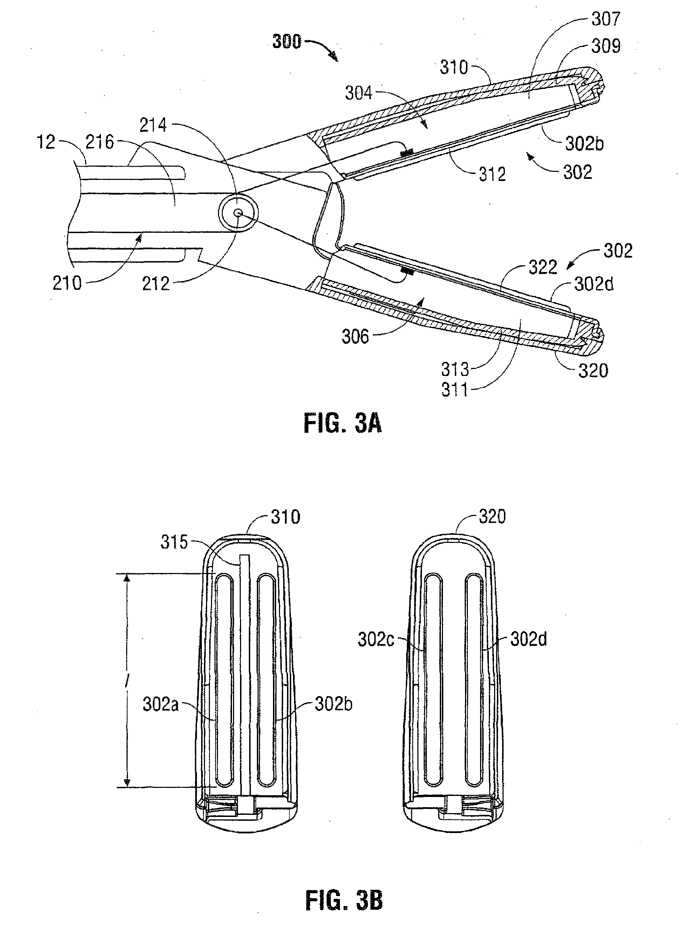

Figs. 3A and 3B illustrate a microwave end effector assembly 300 according to one embodiment of the present disclosure. The end effector assembly 300 is coupled to a coaxial cable 210 that is housed within the shaft 12 and the cable 11. The cable 210 includes an inner conductor 212 surrounded by an inner insulator 214, which is, in turn, surrounded by an outer conductor 216 (e.g., a cylindrical conducting sheath). The inner conductor 212 and outer conductor 216 may be constructed of copper, gold, stainless steel or other conductive metals with similar conductivity values. The metals may be plated with other materials, e.g., other conductive materials, to improve their properties, e.g., to improve conductivity or decrease energy loss, etc.

The end effector assembly 300 includes a microwave antenna assembly 302 having one or more microwave antennas 302a, 302b, 302c and 302d disposed on the sealing surfaces 312 and 322, respectively. In one embodiment, the microwave antennas 302a - 302d may have a length $l$ of about ¼ of the wavelength of the microwave energy being supplied thereto. The microwave antennas 302a - 302d are coupled to the generator 20, which is adapted to supply microwave energy to the forceps 10 through the cable 210. The coaxial cable 210 connects one or more of the microwave antennas 302a - 302d to an active element of the generator 20 through the inner conductor 212 to form a first pole and the remaining microwave antennas 302a - 302d to a ground reference of the generator through the outer conductor 216 to form a second pole.

Fig. 3B shows a top view of the sealing surfaces 312 and 322 with the microwave antennas 302a - 302d configured as longitudinal strips that extend the lengths of the sealing surfaces 312 and 322. The microwave antennas 302a - 302d may be made from any type of conducting, non-reactive metals, such as stainless steel. The microwave antennas 302a - 302d may be configured either in a monopole or dipole arrangement. In a monopolar arrangement, a single microwave antenna, e.g., antenna 302a, is connected to the inner conductor 212 of the cable 210 and is disposed in a respective sealing surface 312.

In a dipole arrangement, two or more microwave antennas, e.g., antennas 302a and 302c may be used. One of the antennas may be the first pole (e.g., coupled to the inner conductor 212 of the cable 210) and another antenna may be a second pole (e.g., coupled to the outer conductor 216 of the cable 210). In one embodiment, the antenna 302a may be the first pole and the antenna 302c may be the second pole, such that the microwave energy flows from the sealing surface 312 to the sealing surface 322. When tissue is sealed by the assembly 300 in this dipole configuration, the antennas 302a and 302c may provide for an automatic termination of the sealing procedure. As sealing progresses, the tissue separating the antennas 302a and 302c is removed, thereby decreasing the separation between the antennas 302a and 302c. As the antennas 302a and 302c are moved toward each other by the compression forces, the microwave energy transmitted therethrough is reflected back therethrough and the radiation automatically stops due to the proximity of the first and second poles (e.g., antennas 302a and 302c).

In another embodiment, the antennas 302a and 302b may be configured as a planar dipole antenna such that the antennas 302a and 302b are disposed side-by-side on the sealing surface 312. More specifically, the antenna 302a may be the first pole and the antenna 302b may be the second pole, such that the energy flows across the sealing surface 112.

In another embodiment, multiple antennas may form the first and second pole, respectively. Any of the two antennas may form the first pole, with the remaining antennas forming the second pole. In particular, antennas 302a and 302b may form the first pole with antennas 302c and 302d forming the second pole, such that microwave energy flows between the sealing surfaces 312 and 322. In another embodiment, the first pole may include the antennas 302a and 302d, while the second pole includes antennas 302b and 302c. Those skilled in the art will appreciate that various other arrangements of antennas 302 are also possible.

The jaw members 310 and 320 also include shielding members 304 and 306 disposed therein, which include respective the sealing surfaces 312 and 322. Each of the shielding members 304 and 306 may include a dielectric portion 307 and 311 and a metallic plate 309 and 313 disposed over the dielectric portions 307 and 311, respectively. The dielectric portions 307 and 311 may be formed from a dielectric material that restricts propagation of microwave energy, such as ceramic. The shielding members 304 and 306, by nature of the relatively high dielectric properties and the presence of the metallic plate, reflect the microwave energy from the antennas 302a - 302d toward tissue being grasped between the sealing surfaces 312 and 322. This arrangement allows for use of any number of antennas 302 (e.g., a single antenna) since the microwave energy is restricted to the volume of tissue being grasped between the jaw members 310 and 320.

The end effector assembly 300 also includes a longitudinally-oriented channel 311 defined in the sealing surface 312 extending from the proximal end to the distal end thereof. The channel 315 facilitates longitudinal reciprocation of the knife 200 along a particular cutting plane to effectively and accurately separate the tissue along a formed tissue seal. The channel 315 may also be defined in the sealing surface 322 or solely disposed in only one sealing surface, e.g., sealing surface 312.

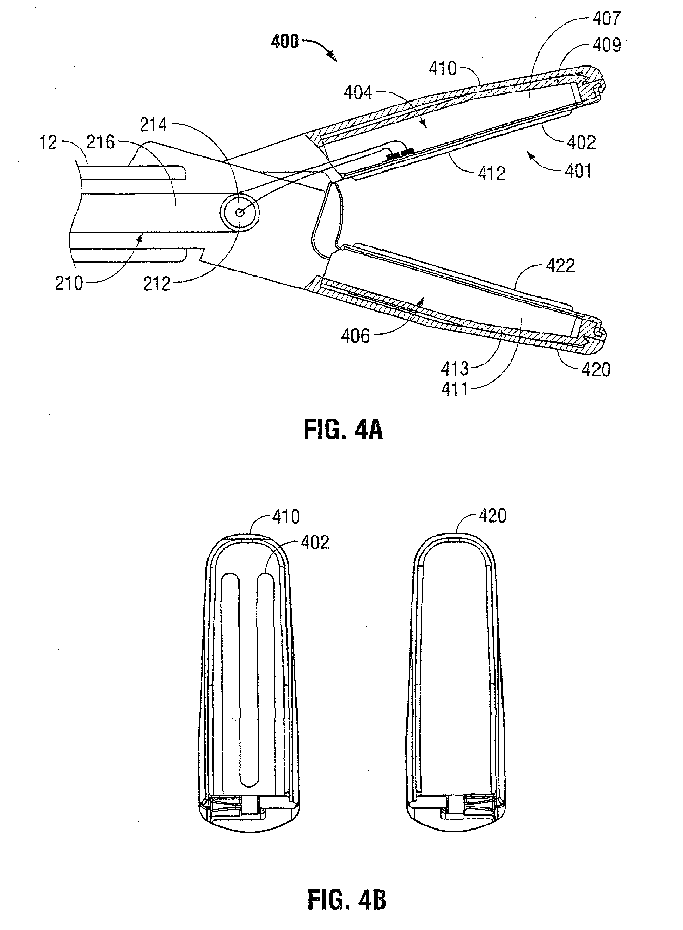

Figs. 4A and 4B illustrate a microwave end effector assembly 400 according to another embodiment of the present disclosure. The end effector assembly 400 includes jaw members 410 and 420 having shielding members 404 and 406 disposed therein, which include sealing surfaces 412 and 422, respectively. Each of the shielding members 404 and 406 may include a respective dielectric portion 407 and 411 and a metallic plate 409 and 413 disposed over the dielectric portions 407 and 411, respectively. The dielectric portions 407 and 411 may be formed from a dielectric material that restricts propagation of microwave energy, such as ceramic. The shielding members 404 and 406, by nature of the relatively high dielectric properties and the presence of the metallic plate, reflect the microwave energy toward tissue being grasped between the sealing surfaces 412 and 422.

The end effector assembly 400 is also coupled to the coaxial cable 210 and includes a microwave antenna assembly 401 having microwave antenna 402 disposed on the sealing surface 412. The microwave antenna 402 may be a so-called "microstrip" antenna, which is embedded in the sealing surface 422 of the shielding member 404. The antenna 402 is wound across the sealing surface 422 to maximize the surface area and the sealing area of the sealing surface 412. As shown in Fig. 4B, the antenna 402 may be wound longitudinally or transversely across the sealing surface 422. The antenna 402 may be a single pole antenna, in which case, the microwave energy is supplied thereto only though one of the conductors of the cable 210. The antenna 402 may be made from any type of conducting non-reactive metals, such as stainless steel. This embodiment, utilising microstrip antennas is not claimed.

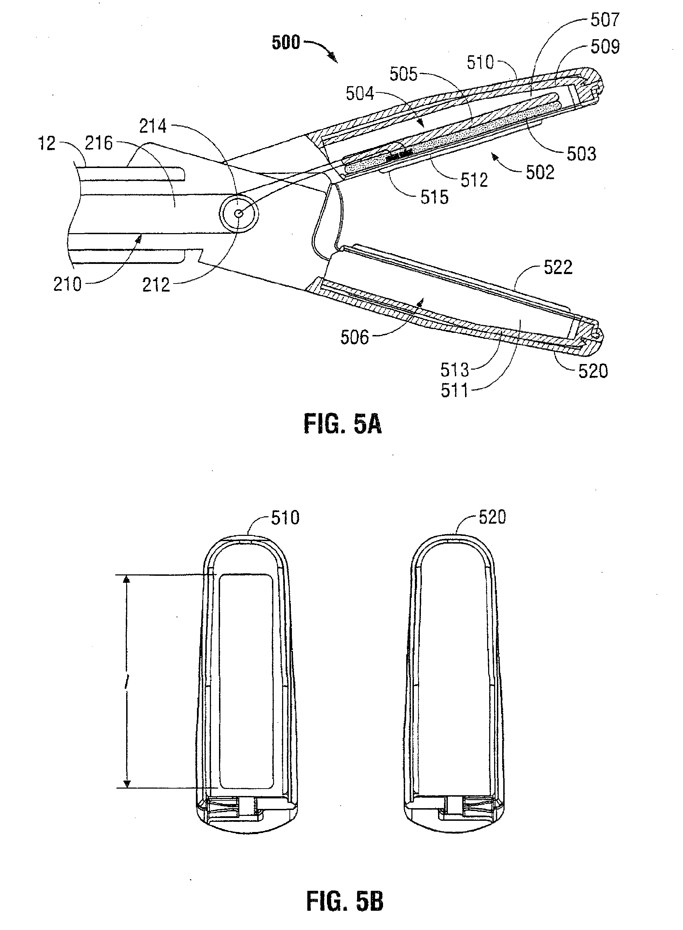

Figs. 5A and 5B illustrate a microwave end effector assembly 500 according to another embodiment of the present disclosure. The end effector assembly 500 includes jaw members 510 and 520 having shielding members 504 and 506 disposed therein, which include sealing surfaces 512 and 522, respectively. Each of the shielding members 504 and 506 may include a respective dielectric portion 507 and 511 and a metallic plate 509 and 513 disposed over the dielectric portions 507 and 511, respectively. The dielectric portions 507 and 511 may be formed from a dielectric material that restricts propagation of microwave energy, such as ceramic. The shielding members 504 and 506, by nature of the relatively high dielectric properties and the presence of the metallic plate, reflect the microwave energy toward tissue being grasped between the sealing surfaces 512 and 522.

The end effector assembly 500 is also coupled to the coaxial cable 210 and includes a microwave antenna assembly 502 disposed on the sealing surface 512. The microwave antenna assembly 502 includes a patch antenna 515 having a substantially rectangular shape. The microwave antenna assembly 502 also includes a dielectric substrate 503 and a grounding member 505. The patch antenna 515 is coupled to the inner conductor 212 of the cable 210 and the grounding member 505 is coupled to the outer conductor 214. The patch antenna 515 and the grounding member 505 are electrically insulated by the substrate 503. The substrate 503 may have a larger surface area than the patch antenna 515 such that the patch antenna 515 is completely covered by the substrate 503 to confine propagation of the microwave energy to the grounding member 505 to the substrate 503. Embodiments referring to patch antennas are not claimed. In another embodiment, the substrate 503 and the grounding member 505 may be replaced by the shielding member 504. In other words, the grounding member 505 may be enclosed within the shielding member 504 and the patch antenna 515 may then be disposed on top thereof. The patch antenna 515 may be made from any type of conducting non-reactive metals, such as stainless steel. The grounding member 505 may be may be constructed of copper, gold, stainless steel or other conductive metals with similar conductivity values. The metals may be plated with other materials, e.g., other conductive materials, to improve their properties, e.g., to improve conductivity or decrease energy loss, etc.

The patch antenna 515 may have a length $l$ that is substantially equal to ½ of the wavelength of the microwave energy being supplied thereto. The wavelength also depends on the dielectric properties of the substrate 503 and/or the shielding member 504. The relationship between the wavelength and the dielectric properties of the materials is expressed by the formula (1):

$$(1)\ \lambda_s = c \mathbin{/} (f \sqrt{\varepsilon_s})$$

wherein c is a constant representing the speed of light, f is the frequency of the microwave energy, and $\varepsilon_s$ is a dielectric permittivity of the substrate 503 and/or the shielding member 504. The formula (1) illustrates that the wavelength $\lambda_s$ may be varied by selecting different frequencies, f, and/or dielectric materials $\varepsilon_s$.

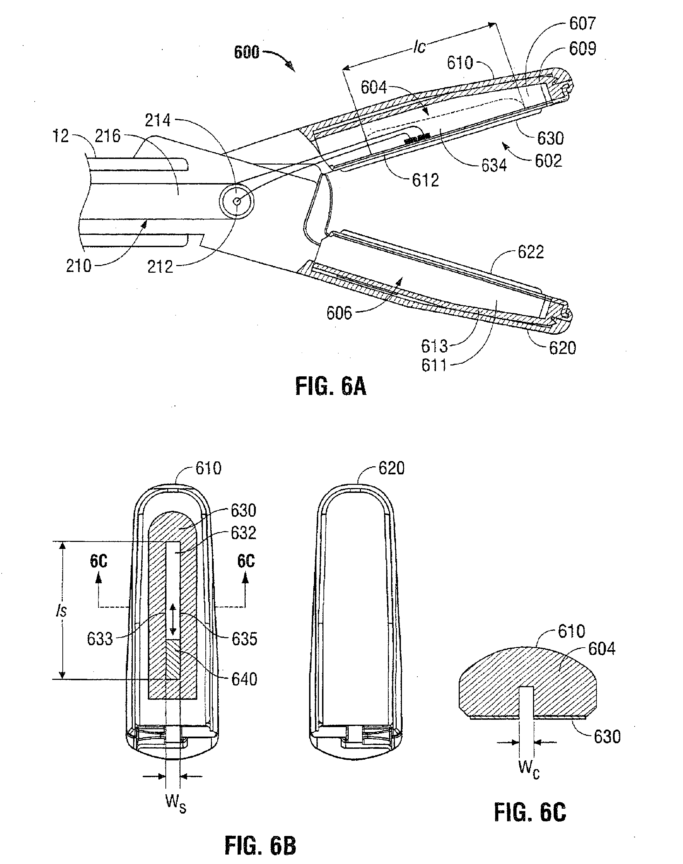

Figs. 6A - 6C illustrate a microwave end effector assembly 600 according to yet another embodiment of the present disclosure. The end effector assembly 600 includes jaw members 610 and 620 having shielding members 604 and 606 disposed therein, which include sealing surfaces 612 and 622, respectively. Each of the shielding members 604 and 606 may include a respective dielectric portion 607 and 611 and a metallic plate 609 and 613 disposed over the dielectric portions 607 and 611, respectively. The dielectric portions 607 and 611 may be formed from a dielectric material that restricts propagation of microwave energy, such as ceramic. The shielding members 604 and 606, by nature of the relatively high dielectric properties and the presence of the metallic plate, reflect the microwave energy toward tissue being grasped between the sealing surfaces 612 and 622.

The end effector assembly 600 is also coupled to the coaxial cable 210 and includes a microwave antenna assembly 602 disposed on the sealing surface 612. The microwave antenna assembly 602 includes a slot antenna 630 having a substantially rectangular slot 632 therein as shown in Fig. 6B. The slot antenna 630 may be made from any type of conducting non-reactive metals, such as stainless steel. The rectangular slot 632 has a length $l_s$ and a width $w_s$.

The microwave antenna assembly 602 may also include a cavity 634 formed within the shielding member 604 of the jaw member 610. In one embodiment, the cavity 634 may extend in a proximal direction to facilitates longitudinal reciprocation of the knife 200 along a particular cutting plane to effectively and accurately separate the tissue along a formed tissue seal. The length $l_c$ and width $w_c$ of the cavity 634 (Figs. 6A and 6C) are substantially equal to the length $l_s$ and the width $w_s$ of the slot 632 (Fig. 6B), such that the slot 632 substantially overlaps the cavity 634. The rectangular slot 632 also includes a first and second longitudinal sides 633 and 635. The first side 633 is coupled to the inner conductor 212 of the cable 210 and the second side 635 is coupled to the outer conductor 214, such that the first side 633 acts as a first pole and the second side 635 acts as a second pole. As microwave energy is supplied to the slot antenna 630, the microwave energy is transmitted from the first side 633 across to the second side 635 and into the cavity 634. In addition, the overlapping of the slot 632 and the cavity 634 allows for directional radiation of microwave energy from the slot antenna 630 toward the jaw member 620 as the microwave energy is bounced downward by the cavity 634. The cavity 634 allows for concentration of the microwave energy down the center of the jaw members 610 and 620, providing for a narrower seal.

The length $l_c$ of the cavity 634 and the length $l_s$ of the slot 632 is substantially equal to ½ of the wavelength of the microwave energy being supplied thereto. The wavelength also depends on the dielectric properties of the surrounding environment and/or the shielding member 604. As microwave energy is applied to the tissue, the tissue is desiccated, which, in turn, changes the dielectric properties of the surrounding environment. Therefore, as illustrated by formula (1) above, based on the relationship between the dielectric permittivity of the surrounding environment, the wavelength of the microwave energy being supplied to the tissue is also affected. Accordingly, to maintain the match between the length $l_c$ of the cavity 634 and the length $l_s$ of the slot 632 and ½ of the wavelength of the microwave energy, the length $l_c$ of the cavity 634 and the length $l_s$ of the slot 632 may be adjusted during operation.

As best shown in Fig. 6B, the antenna assembly 602 includes a retractable plate 640 housed between the shielding member 604 and the slot antenna 630. The retractable plate 640 is movable in a longitudinal direction between the shielding member 604 and the slot antenna 630 such that the opening defined by the slot 632 into the cavity 634 is at least partially covered up. The retractable plate 640 has a width larger than the width $w_s$ of the slot 632, such that when the retractable plate 640 is slid between the shielding member 604 and the slot antenna 630, the retractable plate 640 fully covers the slot 632 up to the point of extension of the retractable plate 640. The length of the retractable plate 640 may be any suitable length, such as the length $l_s$ of the slot 632. This allows for the retractable plate 640 to fully cover the slot 632 when being fully retracted.

During operation, the retraction of the retractable plate 640 may be adjusted to match the antenna assembly 602 to the wavelength of the microwave energy as the dielectric properties of the surrounding media is changing. More specifically, adjusting the length of retraction of the retractable plate 640 adjusts the length $l_c$ of the cavity 634 and the length $l_s$ of the slot 632 to maintain theses lengths substantially equal to ½ of the wavelength of the microwave energy, as the wavelength of the microwave energy is changing due to the changes in the dielectric properties.

The above embodiments of the microwave antenna assemblies may also be utilized to measure certain tissue properties such as temperature and dielectric properties. The microwave antenna assembly is configured to operate in a therapeutic mode to deliver microwave energy to seal tissue and in a detection mode to measure tissue properties.

In one embodiment, the microwave antenna assemblies may be utilized in a receiving mode only. In other words, the antenna assembly may be configured as a radiometer to detect changes in electromagnetic radiation emanating from the tissue. The detected changes in electromagnetic radiation are then processed by the generator 20 to calculate the temperature of the tissue.

In another embodiment, the microwave antenna assemblies may be configured to detect dielectric properties of the tissue. This may be accomplished by transmitting non-therapeutic microwave energy into the tissue and then measuring reflected and forward power. The reflected and forward power is indicative of the dielectric properties of tissue and may be measured based on the impedance mismatch between the generator 20 and tissue due to the changes in dielectric properties of the tissue and other system components. More specifically, impedance mismatches cause a portion of the power, so-called "reflected power," from the generator 20 to not reach the load and cause the power or energy delivered, the so-called "forward power." to vary in an irregular or inconsistent manner over the treatment time interval. The actual power of the generator may be expressed as a sum of the forward power and reflected power. Thus, it is possible to determine the impedance mismatch that is caused by dielectric properties of the tissue by measuring and analyzing the reflected and forward power. This may be accomplished by transmitting a non-therapeutic microwave pulse (e.g., 1 GHz - 5 GHz) and then measuring the reflected and forward power at the generator 20. The generator 20 then accounts for the impedance mismatch caused by system components (e.g., cable and antenna assembly stray capacitance) to calculate the portion of the mismatch due to the dielectric properties of the tissue. This then allows the generator 20 to determine those properties.

**Claims**

1. A microwave forceps (10) for sealing tissue, comprising:

at least one shaft member (12) having an end effector assembly (600) disposed at a distal end thereof, the end effector assembly including opposing jaw members (610, 620) movable from a first position in spaced relation relative to one another to at least one subsequent position wherein the jaw members cooperate to grasp tissue therebetween, each of the jaw members including a sealing surface (612, 622), **characterized by**:

at least the sealing surface of the first jaw member including a microwave antenna assembly (602) couplable to a source of microwave energy (20), wherein the microwave antenna assembly includes a slot antenna (630) having a substantially rectangular slot (632) defined therethrough, the rectangular slot having a first longitudinal side (633) couplable to a ground reference (212) of the microwave energy source and a second longitudinal side (635) couplable to an active element (214) of the microwave energy source.

2. The microwave forceps according to claim 1, further comprising:

a handle assembly (60) including a first handle (62) and a second handle (72), wherein the first handle is movable relative to the second handle; and a pushrod (101) disposed within the at least one shaft, the pushrod operatively coupled at one end to the handle assembly and to the end effector assembly, wherein longitudinal movement of the pushrod moves the jaw members from the first position to the at least one subsequent position.

3. The microwave forceps according to claim 1 or 2, further comprising:

a knife channel (315) defined along a length of at least one of the jaw members, the knife channel dimensioned to reciprocate a cutting mechanism (200) therealong; and an actuator (70) operatively connected to one of the shaft members for selectively advances the cutting mechanism from a first position wherein the cutting mechanism is disposed proximal to tissue held between the jaw members to at least one subsequent position wherein the cutting mechanism is disposed distal to tissue held between the jaw members.

4. The microwave forceps according to claim 1, 2 or 3, wherein the microwave antenna assembly includes at least one monopole microwave antenna.

5. The microwave forceps according to any one of the preceding claims, wherein each of the jaw members includes a shielding member (604, 606) adapted to confine microwave energy between the jaw members.

6. The microwave forceps according to claim 5, wherein the microwave antenna assembly further includes a cavity (634) formed within the shielding member of the first jaw member, wherein the cavity overlaps the rectangular slot.

7. The microwave forceps according to claim 1, wherein the slot (ls) has a length that is substantially equal to about half of a wavelength of the microwave energy being supplied thereto.

8. The microwave forceps according to claim 5, wherein the microwave antenna assembly further includes:

a retractable plate (640) housed between the shielding member of the first jaw member and the slot antenna.

9. The microwave forceps according to claim 5, wherein the microwave antenna assembly further includes:

a retractable plate (640) housed between the shielding member of the first jaw member and the slot antenna, wherein the retractable plate is movable in a longitudinal direction between the shielding member and the slot antenna such that the opening defined by the slot is at least partially covered up.

10. The microwave forceps according to claim 9, wherein the retractable plate is retracted to maintain a length of the rectangular slot that is substantially equal to about half of a wavelength of the microwave energy being supplied thereto.

**Patentansprüche**

1. Mikrowellenzange (10) zum Versiegeln von Gewebe, mit:

mindestens einem Schaftelement (12), das an seinem distalen Ende einen Endeffektoraufbau (600) angeordnet aufweist, wobei der Endeffektoraufbau sich gegenüberstehende Backenelemente (610, 620) aufweist, die von einer ersten zueinander relativ beabstandeten Beziehung zu mindestens einer nachfolgenden Position bewegbar sind, wobei die Backenelemente zusammenwirken, um dazwischen Gewebe zu greifen, und jedes der Backenelemente eine Versiegelungsfläche (612, 622) aufweist, **gekennzeichnet dadurch, dass**

mindestens die Versiegelungsfläche von dem ersten Backenelement einen Mikrowellenantennenaufbau (602) aufweist, der mit einer Mikrowellenenergiequelle (20) koppelbar ist, wobei der Mikrowellenantennenaufbau eine Schlitzantenne (630) mit einem im Wesentlichen rechteckigen Schlitz (632) dort hindurch definiert aufweist und der rechteckige Schlitz eine erste Längsseite (633), die mit einer Referenzmasse (212) der Mikrowellenenergiequelle koppelbar ist und eine zweite Längsseite (635) aufweist, die mit einem aktiven Element (214) der Mikrowellenenergiequelle koppelbar ist.

2. Mikrowellenzange nach Anspruch 1, ferner mit:

einem Griffaufbau (60) einschließlich eines ersten Griffs (62) und eines zweiten Griffs (72), wobei der erste Griff relativ zu dem zweiten Griff bewegbar ist; und
einem Schubstab (101), der in dem mindestens einen Schaft angeordnet ist, wobei der Schubstab betriebsfähig bei einem Ende des Griffaufbaus gekoppelt ist und mit dem Endeffektoraufbau gekoppelt ist, wobei eine Bewegung in Längsrichtung des Stabs die Backenelemente von der ersten Position zu der mindestens einen nachfolgenden Position bewegt.

3. Mikrowellenzange nach Anspruch 1 oder 2, ferner mit:

einem Messerkanal (315), der entlang einer Länge von zumindest einem der Backenelemente definiert ist, wobei der Messerkanal dimensioniert ist, an sich entlang einen Schneidmechanismus (200) hin- und her zu bewegen; und
einem Aktuator (70), der betriebsfähig mit einem der Schaftelemente zum gezielten Vorschieben des Schneidmechanismus von einer ersten Position, in welcher der Schneidmechanismus proximal zu dem zwischen den Backenelementen gehaltenem Gewebe angeordnet ist, zu mindestens einer nachfolgenden Position verbunden ist, wobei der Schneidmechanismus distal zu zwischen den Backenelementen gehaltenem Gewebe angeordnet ist.

4. Mikrowellenzange nach Anspruch 1, 2 oder 3, bei welcher der Mikrowellenantennenaufbau mindestens eine Monopolmikrowellenantenne aufweist.

5. Mikrowellenzange nach einem der vorstehenden Ansprüche, bei der jedes der Backenelemente ein Abschirmelement (604, 606) aufweist, das angepasst ist, Mikrowellenenergie zwischen den Backenelementen zu begrenzen.

6. Mikrowellenzange nach Anspruch 5, bei welcher der Mikrowellenantennenaufbau ferner eine Aussparung (634) aufweist, die in dem Abschirmelement des ersten Backenelements ausgebildet ist, wobei die Aussparung den rechteckigen Schlitz überlappt.

7. Mikrowellenzange nach Anspruch 1, bei welcher der Schlitz (ls) eine Länge aufweist, die im Wesentlichen gleich in etwa der Hälfte der Wellenlänge der Mikrowellenenergie ist, die dorthin geführt wird.

8. Mikrowellenzange nach Anspruch 5, bei welcher der Mikrowellenantennenaufbau ferner aufweist:

   eine rückziehbare Platte (640), die zwischen dem Abschirmelement des ersten Backenelements und der Schlitzantenne aufgenommen ist.

9. Mikrowellenzange nach Anspruch 5, bei welcher der Mikrowellenantennenaufbau ferner aufweist:

   eine rückziehbare Platte (640), die zwischen dem Abschirmelement des ersten Backenelements und der Schlitzantenne aufgenommen ist, wobei die rückziehbare Platte in einer Längsrichtung zwischen dem Abschirmelement und der Schlitzantenne bewegbar ist, sodass die durch den Schlitz definierte Öffnung zumindest teilweise zugedeckt ist.

10. Mikrowellenzange nach Anspruch 9, bei der die rückziehbare Platte zurückgezogen ist, um eine Länge des rechteckigen Schlitzes aufrechtzuerhalten, die im Wesentlichen gleich der Hälfte einer Wellenlänge der Mikrowellenenergie ist, die dort hingeführt wird.

**Revendications**

1. Forceps à micro-ondes (10) pour le scellement de tissus, comprenant :

   au moins un élément d'arbre (12) ayant un ensemble d'effecteur terminal (600) disposé à une extrémité distale de celui-ci, l'ensemble effecteur terminal incluant des éléments de mâchoire opposés (610, 620) déplaçables d'une première position, en une relation espacée l'un de l'autre, à au moins une position suivante dans laquelle les éléments de mâchoire coopèrent pour saisir le tissu entre eux, chacun des éléments de mâchoire incluant une surface de scellement (612, 622), **caractérisé par** :

   au moins la surface de scellement du premier élément de mâchoire incluant un ensemble d'antenne à micro-ondes (602) apte à être couplé à une source d'énergie micro-onde (20) lorsque l'ensemble d'antenne à micro-ondes inclut une antenne à fente (630) ayant une fente (632) sensiblement rectangulaire définie à travers celle-ci, la fente rectangulaire ayant un premier côté longitudinal (633) apte à être couplé à une référence de masse (212) de la source d'énergie micro-onde et un second côté longitudinal (635) apte à être couplé à un élément actif (214) de la source d'énergie micro-onde.

2. Forceps à micro-ondes selon la revendication 1, comprenant en outre :

   un ensemble de poignées (60) incluant une première poignée (62) et une seconde poignée (72), où la première poignée est déplaçable relativement à la seconde poignée, et une tige de poussée (101) disposée dans le au moins un arbre, la tige de poussée étant fonctionnellement couplée à une extrémité à l'ensemble de poignée et à l'ensemble d'effecteur terminal, où un mouvement longitudinal de la tige de poussée déplace les éléments de mâchoire de la première position à ladite au moins une position suivante.

3. Forceps à micro-ondes selon la revendication 1 ou 2, comprenant en outre :

   un canal de couteau (315) défini le long d'une longueur d'au moins un des éléments de mâchoire, le canal de couteau étant dimensionné pour un mouvement alternatif d'un mécanisme de coupe (200) le long de celui-ci ; et un actionneur (70) fonctionnellement connecté à un des éléments de mâchoire pour faire avancer sélectivement le mécanisme de coupe d'une première position dans laquelle le mécanisme de coupe est disposé d'une manière proximale au tissu tenu entre les éléments de mâchoire à au moins une position suivante dans laquelle le mécanisme de coupe est disposé d'une manière distale au tissu tenu entre les éléments de mâchoire.

**4.** Forceps à micro-ondes selon la revendication 1, 2 ou 3, dans lequel l'ensemble d'antenne à micro-ondes inclut au moins une antenne à micro-ondes unipolaire.

**5.** Forceps à micro-ondes selon l'une quelconque des revendications précédentes, dans lequel chacun des éléments de mâchoire inclut un élément de protection (604, 606) apte à confiner l'énergie micro-onde entre les éléments de mâchoire.

**6.** Forceps à micro-ondes selon la revendication 5, dans lequel l'ensemble d'antenne à micro-ondes inclut en outre une cavité (634) formée dans l'élément de protection du premier élément de mâchoire, où la cavité chevauche la fente rectangulaire.

**7.** Forceps à micro-ondes selon la revendication 1, dans lequel la fente (ls) a une longueur qui est sensiblement égale à environ la moitié d'une longueur d'onde de l'énergie micro-onde fournie à celle-ci.

**8.** Forceps à micro-ondes selon la revendication 5, dans lequel l'ensemble d'antenne à micro-ondes comprend en outre :

une plaque rétractable (640) logée entre l'élément de protection du premier élément de mâchoire et l'antenne à fente.

**9.** Forceps à micro-ondes selon la revendication 5, dans lequel l'ensemble d'antenne à micro-ondes comprend en outre :

une plaque rétractable (640) logée entre l'élément de protection du premier élément de mâchoire et l'antenne à fente, où la plaque rétractable est déplaçable dans une direction longitudinale entre l'élément de protection et l'antenne à fente de telle sorte que l'ouverture définie par la fente est au moins partiellement recouverte.

**10.** Forceps à micro-ondes selon la revendication 9, dans lequel la plaque rétractable est rétractée pour maintenir une longueur de la fente rectangulaire qui est sensiblement égale à environ la moitié d'une longueur d'onde de l'énergie micro-onde fournie à celle-ci.

**FIG. 1**

EP 2 233 098 B1

**FIG. 2**

EP 2 233 098 B1

**FIG. 3A**

**FIG. 3B**

FIG. 4A

FIG. 4B

**FIG. 5A**

**FIG. 5B**

**FIG. 6A**

**FIG. 6B**

**FIG. 6C**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 6152923 A